(19) Europäisches Patentamt
European Patent Office
Office européen des brevets

(11) Veröffentlichungsnummer: **0 197 280**
**B1**

# (12) EUROPÄISCHE PATENTSCHRIFT

(45) Veröffentlichungstag der Patentschrift: **07.06.89**

(51) Int. Cl.⁴: **C07C 127/22**, C07C 157/12, A01N 47/34

(21) Anmeldenummer: **86102490.9**

(22) Anmeldetag: **26.02.86**

## (54) Benzoylharnstoffe.

(30) Priorität: **09.03.85 DE 3508429**

(43) Veröffentlichungstag der Anmeldung: **15.10.86 Patentblatt 86/42**

(45) Bekanntmachung des Hinweises auf die Patenterteilung: **07.06.89 Patentblatt 89/23**

(84) Benannte Vertragsstaaten: **AT BE CH DE FR GB IT LI NL**

(56) Entgegenhaltungen:
**EP-A- 0 035 084**
**EP-A- 0 052 833**

**Die Akte enthält technische Angaben, die nach dem Eingang der Anmeldung eingereicht wurden und die nicht in dieser Patentschrift enthalten sind.**

(73) Patentinhaber: **BAYER AG, D-5090 Leverkusen 1 Bayerwerk(DE)**

(72) Erfinder: **Sirrenberg, Wilhelm, Dr., Wuppertaler Strasse 21, D-4322 Sprockhövel(DE)**
Erfinder: **Klauke, Erich, Dr., Eichendorffweg 8, D-5068 Odenthal(DE)**
Erfinder: **Becker, Benedikt, Dr., Metzkausener Strasse 14, D-4020 Mettmann(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft neue substituierte 1-Phenyl-3-benzoyl-(thio)harnstoffe, mehrere Verfahren zu ihrer Herstellung und ihre Verwendung als Schädlingsbekämpfungsmittel, insbesondere als Insektizide und Akarizide.

Es ist bereits bekannt, daß bestimmte Benzoylharnstoffe, wie z. B. 1-(3,5-Dichlor-2,4-difluorphenyl)-3-(2,6-difluor-benzoyl)-harnstoff insektizide Eigenschaften besitzen (vgl. z. B. EP-A-52 833 und EP-A- 35 084).

Es wurden die neuen substituierten 1-Phenyl-3-benzoyl-(thio)harnstoffe der allgemeinen Formel (I)

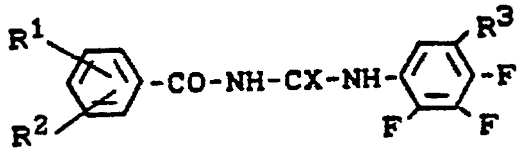
(I)

gefunden, in welcher
X für Sauerstoff oder Schwefel steht,
$R^1$ und $R^2$ gleich oder verschieden sind und für Wasserstoff, Halogen oder Alkyl stehen und
$R^3$ für Halogen steht,
wobei wenn
$R^1$ für 2-Fluor oder 2-Chlor steht,
$R^2$ für Wasserstoff, 6-Fluor oder 6-Chlor steht und
$R^3$ für Fluor steht,
in den Fällen
X für Schwefel steht.

Diese neuen Verbindungen weisen starke biologische, insbesondere insektizide Eigenschaften auf, die ihre Verwendung als Schädlingsbekämpfungsmittel, insbesondere als Insektizide und Akarizide ermöglichen.

Weiterhin wurde gefunden, daß die neuen substituierten 1-Phenyl-3-benzoyl-(thio)harnstoffe der allgemeinen Formel (I) erhalten werden, indem man

a) substituierte Aniline der allgemeinen Formel (II)

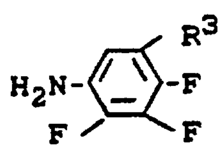
(II)

in welcher
$R^3$ die oben angegebene Bedeutung hat,
mit Benzoyl-iso(thio)cyanaten der allgemeinen Formel (III)

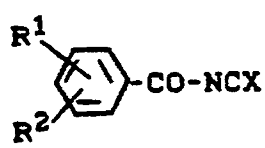
(III)

in welcher
X, $R^1$ und $R^2$ die oben angegebene Bedeutung haben,
gegebenenfalls in Gegenwart eines Verdünnungsmittels umsetzt oder

b) substituierte Phenyl-iso(thio)cyanate der allgemeinen Formel (IV)

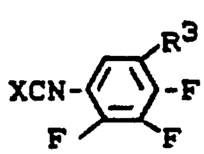
(IV)

in welcher
X und $R^3$ die oben angegebenen Bedeutungen haben,
mit Benzoesäureamiden der allgemeinen Formel (V)

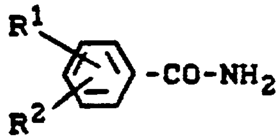

(V)

in welcher
$R^1$ und $R^2$ die oben angegebenen Bedeutungen haben,
gegebenenfalls in Gegenwart eines Katalysators und gegebenenfalls in Gegenwart eines Verdünnungsmittels umsetzt.

Die Alkylreste $R^1$ und $R^2$ können gleich oder verschieden sein und bedeuten geradkettiges oder verzweigtes Alkyl mit 1 bis 12, vorzugsweise 1 bis 6, insbesondere 1 bis 4 und ganz besonders bevorzugt 1 oder 2 Kohlenstoffatomen. Beispielhaft seien Methyl, Ethyl, n-Propyl, i-Propyl, n-Butyl, sec.-Butyl, i-Butyl und tert.-Butyl genannt.

Besonders bevorzugt wird die Methylgruppe.

Halogen bedeutet in der Definition von $R^1$, $R^2$ und $R^3$ Fuor, Chlor, Brom und Iod, vorzugsweise Fluor, Chlor und Brom, insbesondere Fluor und Chlor.

Vorzugsweise ist in den neuen Verbindungen wenigstens einer der Reste $R^1$ und $R^2$ Wasserstoff verschieden.

$R^1$ steht vorzugsweise in der 2-Position, $R^2$ vorzugsweise in der 4-Position, 5-Position oder in der 6-Position im Phenylring des Benzoylteils, wobei $R^2$ sich besonders bevorzugt in der 4-, 5- oder 6-Position befindet, wenn X für Schwefel steht und $R^2$ sich besonders bevorzugt in der 4- oder 5-Position (insbesondere 4-Position) befindet, wenn X für Sauerstoff steht.

Die neuen Verbindungen der allgemeinen Formel (I) verfügen über Eigenschaften, die ihre Verwendung als Schädlingsbekämpfungsmittel ermöglichen, insbesondere zeichnen sie sich sowohl durch eine hervorragende und langanhaltende insektizide als auch akarizide Wirksamkeit aus. Sie unterscheiden sich hierdurch von den bekannten Benzoylharnstoffen, für welche eine insektizide Wirksamkeit bekannt ist.

Die Erfindung betrifft vorzugsweise neue Verbindungen der allgemeinen Formel (I), in welcher X für Sauerstoff oder Schwefel steht,
$R^1$ und $R^2$ gleich oder verschieden sind und für Wasserstoff, Halogen oder $C_1$-$C_6$-Alkyl stehen und
$R^3$ für Halogen steht,
wobei wenn
$R^1$ für 2-Fluor oder 2-Chlor steht,
$R^2$ für Wasserstoff, 6-Fluor oder 6-Chlor steht und
$R^3$ für Fluor steht,
in den Fällen
X für Schwefel steht.

Besonders bevorzugt sind die Verbindungen der allgemeinen Formel (I), in welcher
X für Sauerstoff oder Schwefel steht,
$R^1$ und $R^2$ gleich oder verschieden sind und für Wasserstoff, Halogen oder $C_1$-$C_4$-Alkyl stehen und
$R^3$ für Halogen steht,
wobei, wenn
$R^1$ für 2-Fluor oder 2-Chlor steht,
$R^2$ für Wasserstoff, 6-Fluor oder 6-Chlor steht und
$R^3$ für Fluor steht,
in den Fällen
X für Schwefel steht.

Ganz besonders bevorzugt sind die Verbindungen der allgemeinen Formel (I), in welcher
X für Sauerstoff oder Schwefel steht,
$R^1$ für Fluor, Chlor, Brom oder Methyl steht,
$R^2$ für Wasserstoff, Fluor oder Chlor steht und
$R^3$ für Fluor, Chlor oder Brom steht,
wobei wenn
$R^1$ für 2-Fluor oder 2-Chlor steht,
$R^2$ für Wasserstoff, 6-Fluor oder 6-Chlor steht und
$R^3$ für Fluor steht,
in den Fällen
X für Schwefel steht.

Eine besonders gute Wirksamkeit weisen Verbindungen der allgemeinen Formel (I) auf, in welcher
X für Schwefel steht,
$R^1$ in der 2-Position ist und für Fluor, Chlor, Brom oder Methyl steht,
$R^2$ für Wasserstoff oder für Fluor oder Chlor in der 4-, 5- oder 6-Position steht und
$R^3$ für Fluor oder Chlor steht,
oder in welcher
X für Sauerstoff steht,
$R^1$ in der 2-Position ist und für Fluor, Chlor oder Brom steht,
$R^2$ in der 4- oder 5-Position ist und für Fluor oder Chlor steht und
$R^3$ für Fluor oder Chlor steht.

Verwendet man gemäß Verfahrensvariante (a) 2,4-Difluorbenzoylisothiocyanat und 2,3,4,5-Tetrafluoranilin als Ausgangsstoffe, so kann der Reaktionsablauf durch das folgende Formelschema wiedergegeben werden:

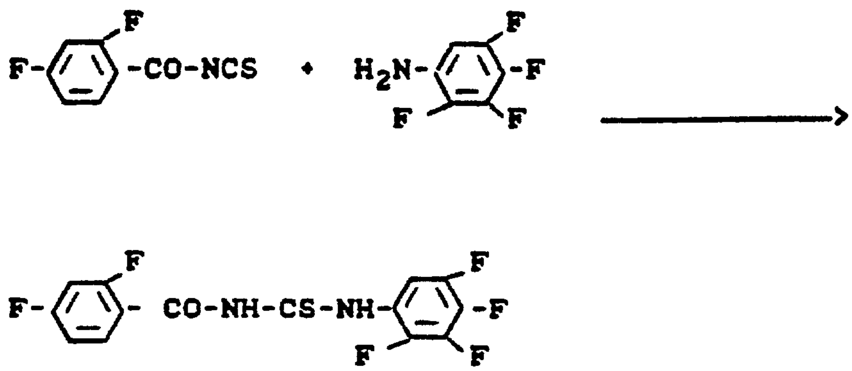

Verwendet man nach Verfahrensvariante (b) 5-Chlor-2,3,4-trifluor-phenyl-isocyanat und 2,6-Difluorbenzamid als Ausgangsstoffe, so kann der Reaktionsablauf durch das folgende Formelschema wiedergegeben werden:

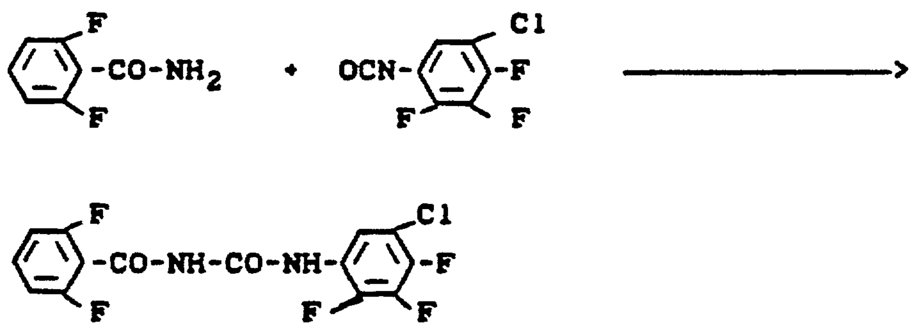

Die Ausgangsverbindungen der Formel (II) sind bekannt oder können nach allgemein bekannten Methoden erhalten werden (vgl. z.B. EP-A- 35 084).

Als Beispiele für die Verbindungen der Formel (II) seien genannt: 2,3,4,5-Tetrafluor-, 5-Chlor-2,3,4-trifluor- und 5-Brom-2,3,4-trifluor-anilin.

Die Ausgangsverbindungen der allgemeinen Formel (III) sind bekannt oder nach allgemein bekannten Methoden erhältlich.

Als Beispiele für die Verbindungen der Formel (III) seien genannt: 2-Fluor-, 2-Chlor-, 2-Brom-, 2-Methyl-, 2,6-Difluor-, 2,6-Dichlor-, 2-Chlor-6-fluor-, 2-Chlor-4-fluor, 2,4-Difluor-, 2,4-Dichlor-, 2,5-Difluor-, 2,5-Dichlor-, 2-Chlor-5-fluor-, 4-Fluor-, 4-Chlor-, 4-Brom- und 4-Methylbenzoylisocyanat bzw. -isothiocyanat.

Die Ausgangsverbindungen der allgemeinen Formel (IV) sind bekannt oder die Aminogruppe der Verbindungen der Formel (II) kann nach üblichen Verfahren in die Isocyanat- bzw. Iso-thio-cyanat-Gruppe umgewandelt werden, z. B. durch Umsetzung mit Phosgen bzw. Thiophosgen in Verdünnungsmitteln wie z.B. Toluol und/oder Pyridin, bei einer Temperatur zwischen -20°C bis +50°C.

Als Beispiele für die Verbindungen der Formel (IV) seien genannt:
2,3,4,5-Tetrafluor-, 5-Chlor-2,3,4-trifluor- und 5-Brom-2,3,4-trifluor-phenylisocyanat bzw. -phenylisothiocyanat.

Die Ausgangsverbindungen der allgemeinen Formel (V) sind ebenfalls bekannt oder nach allgemein bekannten Methoden erhältlich.

Als Beispiele für die Verbindungen der Formel (V) seien genannt: 2-Fluor-, 2-Chlor-, 2-Brom-, 2-Methyl-, 2,6-Difluor-, 2,6-Dichlor-, 2-Chlor-6-fluor-, 2-Chlor-4- fluor-, 2,4-Difluor-, 2,4-Dichlor-, 2,5-Difluor-, 2,5-Dichlor-, 2-Chlor-5-fluor-, 4-Fluor-, 4-Chlor-, 4-Brom- und 4-Methyl-benzoesäureamid.

Als Verdünnungsmittel kommen bei der Durchführung der Verfahrensvarianten (a) und (b) praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone, wie Aceton, Methylethyl-,Methylisopropyl- und Methylisobutylketon, Ester, wie Essigsäuremethylester und -ethylester, Nitrile, wie z.B. Acetonitril und Propionitril, Amide, wie z.B. Dimethylacetamid und N-Methyl-pyrrolidon, sowie Tetramethylensulfon.

Als Katalysatoren können für die Umsetzuung gemäß Verfahrensvariante (b) vorzugsweise tertiäre Amine, wie Triethylamin und 1,4-Diazabicyclo[2,2,2]-octan, sowie organische Zinn-Verbindungen, wie z. B. Dibutylzinndilaurat verwendet werden. Der Zusatz solcher Katalysatoren ist jedoch nicht erforderlich.

Die Reaktionstemperatur kann bei den Verfahrensvarianten (a) und (b) innerhalb eines größeren Bereiches variiert werden. Im allgemeinen arbeitet man bei der Verfahrensvariante (a) zwischen 20 und 180°C, vorzugsweise zwischen 40 und 120°C und bei der Verfahrensvariante (b) zwischen 20 und 200°C, vorzugsweise zwischen 60 und 190°C. Die erfindungsgemäßen Verfahrensvarianten werden im allgemeinen bei Normaldruck durchgeführt.

Zur Durchführung der erfindungsgemäßen Verfahrensvarianten werden die Ausgangsstoffe gewöhnlich in etwa äquimolaren Mengen eingesetzt. Ein Überschuß der einen oder anderen Reaktionskomponenten bringt keine wesentlichen Vorteile.

Die Aufarbeitung der Reaktionsprodukte geschieht nach üblichen Methoden, z.B. durch Absaugen des ausgefallenen Produktes oder durch Herauslösen von unerwünschten Nebenprodukten aus dem Reaktionsgemisch. Zur Charakterisierung dient der Schmelzpunkt.

Die Wirkstoffe der allgemeinen Formel (I) eignen sich bei guter Pflanzenverträglichkeit und günstiger Warmblütertoxizität zur Bekämpfung von tierischen Schädlingen, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec..

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp..

Aus der Ordnung der Mallophaga z.B. Trichodectes spp., Damalinea spp..

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp..

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Erisoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilparvata lugens, Aonidiella aurantii, Aspidiotus hederae, Pseudococcus spp., Psylla spp..

Aus der Ordnung der Lepidoptera z.B. Pectinophera gossypiella, Bupalus piniarius, Cheimatobia brumata, Lithocolletis blancardella, Hyponomeuta padella, Plutella maculipennis, Malacosoma neustria, Euproctis chryssorhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenes spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstritialis, Cotelytra zealandica.

Aus der Ordnung der Hymenoptera z.B. Diprion spp., Hoplocampa spp., Lasius spp., Monomorium pharaonis, Vespa spp..

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xeropsylla cheopis, Ceratophyllus spp..

Aus der Ordnung der Arachnida z. B. Scorpio maurus, Latrodectus mactans.

Aus der Ordnung der Acarina z. B. Acarus siro, Argas spp., Ornithodores spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe der Formel (I) zeichnen sich durch eine hervorragende insektizide und akarizide Wirksamkeit aus. Sie zeigen insbesondere beim Einsatz als Insektizide eine hervorragende Wirkung und eine besonders lange Wirksamkeit gegen Raupen, wie z.B. Plutella maculipennis. Die neuen Verbindungen zeigen außerdem eine fungizide Wirkung gegen Pyricularia oryzae am Reis.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstofen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetisch Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: - z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeine zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die Wirksamkeit der erfindungsgemäßen Verbindungen der allgemeinen Formel (I) soll anhand der folgenden biologischen Beispiele erläutert werden:

Beispiel: A

Heliothis armigera - Test Lösungsmittel: 15 Gewichtsteile Dimethylformamid
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) werden durch Tauchen in die Wirkstoffzubereitung der gewünschten Konzentration behandelt und mit dem Baumwollkapselwurm (Heliothis armigera) besetzt, solange die Blätter noch feucht sind.

Nach der gewünschten Zeit wird die Abtötung in % bestimmt. Dabei bedeutet 100%, daß alle Raupen abgetötet wurden; 0% bedeutet, daß keine Raupen abgetötet wurden.

Bei diesem Test zeigen z.B. bei einer Wirkstoffkonzentration von 0,1% die folgenden Verbindungen der Herstellungsbeispiele (6), (7), (10) und (12) nach 7 Tagen eine Abtötung von 100%.

Beispiel B

Plutella-Test (Wirkungsdauer nach Angießen) Lösungsmittel: 15 Gewichtsteile Dimethylformamid
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit je 50 ml Wirkstoffzubereitung der gewünschten Konzentration werden Kohlpflanzen (Brassica oleracea) angegossen, so daß die Wirkstoffzubereitung in den Boden eindringt, ohne den Sproß zu benetzen. Der Wirkstoff wird von den Wurzeln aufgenommen und in den Sproß weitergeleitet.

Nach der gewünschten Zeit werden die Pflanzen mit Raupen der Kohlschabe (Plutella maculipennis) besetzt. Nach weiteren 7 Tagen wird die Abtötung in % bestimmt. Dabei bedeutet 100%, daß alle Raupen abgetötet wurden; 0% bedeutet, daß keine Raupen abgetötet wurden.

Bei diesem Test zeigen z.B. bei einer Wirkstoffkonzentration von 0,025%, die folgenden Verbindungen der Herstellungsbeispiele (2), (3), (4) und (13) nach 25 Tagen eine Abtötung von 90-100%.

Beispiel: C

Tetranychus-Test (resistent) Lösungsmittel: 15 Gewichtsteile Dimethylformamid
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100%, daß alle Spinnmilben abgetötet wurden; 0 % bedeutet, daß keine Spinnmilben abgetötet wurden.

Bei diesem Test zeigen z.B. bei einer Wirkstoffkonzentration von 0,1 %, die folgenden Verbindungen der Herstellungsbeispiele (3) und (19) nach 10 Tagen eine Abtötung von nahezu 100 % (ermittelter Wert 98 %).

Die Herstellung der erfindungsgemäßen Verbindungen soll durch die folgenden Herstellungsbeispiele erläutert werden.

Beispiel 1:

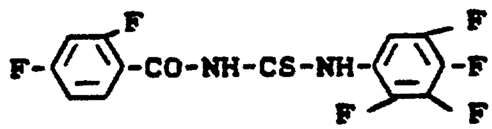

(Verfahrensvariante, (a))

Zu einer Lösung von 1,65g (0,01 Mol) 2,3,4,5-Tetrafluoranilin in 40 ml trockenem Toluol wird eine Lösung von 1,99g (0,01 Mol) 2,4-Difluorbenzoylisothiocyanat in 10 ml trockenem Toluol gegeben, eine halbe Stunde bei 80°C gerührt und anschließend im Vakuum eingeengt. Durch Zugabe von Petrolether wird die Verbindung ausgefällt, anschließend abgesaugt und getrocknet. Man erhält 2,8 g (7% der Theorie) 1-(2,4-Difluorbenzoyl)-3-(2,3,4,5-tetrafluorphenyl)-thioharnstoff vom Schmelzpunkt 126°C.

Beispiel 2:

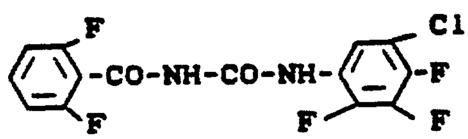

(Verfahren (b))

Zu 3,14 g (0,02 Mol) 2,6-Difluorbenzamid in 14 ml trockenem Dimethylformamid werden 4,57g (0,022 Mol) 5-Chlor-2,3,4-trifluorphenylisocyanat gegeben und 10 Stunden bei 100°C gerührt. Nach Abkühlen auf 20°C wird mit Methanol oder wäßrigem Methanol versetzt, wobei das Produkt ausfüllt. Anschließend wird das Produkt abgesaugt, mit kaltem Methanol abgedeckt und dann getrocknet. Man erhält 6,8g (93 % der Theorie) 1-(2,6-Difluorbenzoyl)-3-(5-chlor-2,3,4-trifluorphenyl)-harnstoff vom Schmelzpunkt 226°C.

Analog Beispiel 1 und 2 bzw. Verfahrensvariante (a) oder (b) können die in der nachfolgenden Tabelle 1 aufgeführten Verbindungen der Formel (I) hergestellt werden:

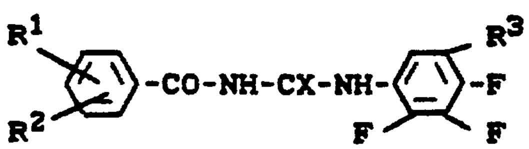 (I)

Tabelle: 1

Tabelle 1

| Beispiel Nr. | $R^1$ | $R^2$ | X | $R^3$ | Schmelzpunkt (°C) |
|---|---|---|---|---|---|
| 3 | 2-F | 6-F | S | F | 127 |
| 4 | 2-F | 6-F | S | Cl | 151 |
| 5 | 2-Cl | 6-F | O | Cl | 248 |
| 6 | 2-Cl | 4-F | O | Cl | 215 |
| 7 | 2-Cl | 4-F | O | F | 180 |
| 8 | 2-Cl | 6-F | S | Cl | 191 |
| 9 | 2-Cl | 6-F | S | F | 169 |
| 10 | 2-Cl | 4-F | S | Cl | 134 |
| 11 | 2-Cl | 5-F | S | Cl | 119 |
| 12 | 2-Cl | H | O | Cl | 210 |
| 13 | 2-Br | H | S | Cl | 130 |
| 14 | 2-$CH_3$ | H | O | Cl | 226 |
| 15 | 2-Cl | H | S | F | 107 |
| 16 | 2-Cl | 6-Cl | S | F | 194 |
| 17 | 2-$CH_3$ | H | S | F | 117 |
| 18 | 2-Cl | 5-F | S | F | 119 |
| 19 | 2-Cl | 4-F | S | F | 113 |
| 20 | 2-Br | H | S | F | 148 |
| 21 | 2-$CH_3$ | H | S | Cl | 149 |
| 22 | 2-Cl | 6-Cl | S | Cl | 211 |
| 23 | H | H | S | F | 139 |
| 24 | 2-F | H | S | F | 140 |
| 25 | H | H | S | Cl | 149 |
| 26 | 2-F | H | S | Cl | 127 |
| 27 | 2-F | 4-F | S | Cl | 138 |
| 28 | 2-F | 4-F | O | F | 181 |
| 29 | 2-F | 4-F | O | Cl | 181 |
| 30 | 3-Cl | 4-Cl | O | Cl | 242 |
| 31 | 3-Cl | 4-Cl | O | F | 230 |
| 32 | 4-Cl | H | O | Cl | 246 |
| 33 | 4-Cl | H | O | F | 233 |

## Patentansprüche

1. Verbindungen der allgemeinen Formel (I)

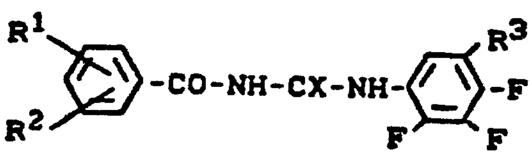

(I)

in welcher
X für Sauerstoff oder Schwefel steht,
$R^1$ und $R^2$ gleich oder verschieden sind und für Wasserstoff, Halogen oder Alkyl stehen und
$R^3$ für Halogen steht,
wobei wenn

$R^1$ für 2-Fluor oder 2-Chlor steht,
$R^2$ für Wasserstoff, 6-Fluor oder 6-Chlor steht und
$R^3$ für Fluor steht,
in den Fällen
X für Schwefel steht.

2. Verbindungen der allgemeinen Formel (I) gemäß Anspruch 1, in welcher $R^1$ Wasserstoff oder für Halogen oder Alkyl in der 2-Position und $R^2$ für Wasserstoff oder für Halogen oder Alkyl in der 4- oder 5-Position des Benzoylphenylringes und X für Sauerstoff stehen.

3. Verbindungen der allgemeinen Formel (I) gemäß Anspruch 1, in welcher $R^1$ für Wasserstoff oder für Halogen oder Alkyl in der 2-Position and $R^2$ für Wasserstoff, Halogen oder Alkyl in der 4-, 5- oder 6-Position des Benzoylphenylringes and X für Schwefel stehen.

4. Verbindungen gemäss Anspruch 1, wobei Alkyl $C_1$–$C_6$-Alkyl bedeutet.

5. Verbindungen gemäss Anspruch 1, wobei Alkyl $C_1$–$C_4$-Alkyl bedeutet.

6. Verfahren zur Herstellung von Verbindungen gemäss Anspruch 1, dadurch gekennzeichnet, dass man

a) substituierte Aniline der allgemeinen Formel (II)

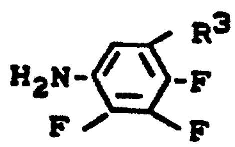
(II)

mit Benzoyl-iso(thio)cyanaten der allgemeinen Formel (III)

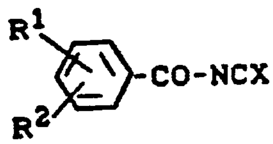
(III)

wobei
X, $R^1$, $R^2$ und $R^3$die in Anspruch 1 angegebene Bedeutung haben,
gegebenenfalls in Gegenwart eines Verdünnungsmittels umsetzt oder
b) substituierte Phenyl-iso(thio)cyanate der allgemeinen Formel (IV)

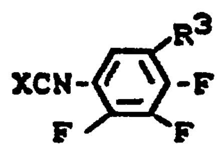
(IV)

mit Benzoesäureamiden der allgemeinen Formel (V)

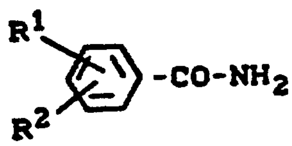
(V)

wobei X, $R^1$, $R^2$ und $R^3$die im Anspruch 1 angegebenen Bedeutungen haben,
gegebenenfalls in Gegenwart eines Katalysators und gegebenenfalls in Gegenwart eines Verdünnungsmittels umsetzt.

7. Schädlingsbekämpfungsmittel, gekennzeichnet durch einen Gehalt an mindestens einer Verbindung der Formel (I) gemäß Anspruch 1 oder 6.

8. Verwendung von Verbindungen der Formel (I) gemäß Anspruch 1 oder 6 zur Bekämpfung von Schädlingen, insbesondere Insekten und Akariden.

9. Verfahren zur Bekämpfung von Schädlingen, dadurch gekennzeichnet, daß man Verbindungen der Formel (I) gemäß Anspruch 1 oder 6 auf die Schädlinge, vorzugsweise Insekten oder Akariden oder ihren Lebensraum einwirken läßt.

10. Verfahren zur Herstellung von Schädlingsbekämpfungsmitteln, dadurch gekennzeichnet, daß man Verbindungen der Formel (I) gemäß Anspruch 1 oder 6 mit Streckmitteln und/oder oberflächenaktiven Mitteln vermischt.

## Claims

1. Compounds of the general formula (I)

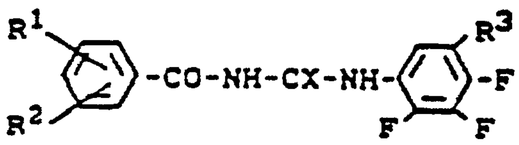

(I)

in which
X represents oxygen or sulphur,
$R^1$ and $R^2$ are identical or different and represent hydrogen, halogen or alkyl and
$R^3$ represents halogen,
and wherein, if
$R^1$ represents 2-fluoro or 2-chloro,
$R^2$ represents hydrogen, 6-fluoro or 6-chloro and
$R^3$ represents fluorine,
in those cases
X represents sulphur.

2. Compounds of the general formula (I) according to Claim 1, in which $R^1$ represents hydrogen or represents halogen or alkyl in the 2-position and $R^2$ represents hydrogen or represents halogen or alkyl in the 4- or 5-position of the benzoylphenyl ring, and X represents oxygen.

3. Compounds of the general formula (I) according to Claim 1, in which $R^1$ represents hydrogen or represents halogen or alkyl in the 2-position and $R^2$ represents hydrogen, halogen or alkyl in the 4-, 5- or 6-position of the benzoylphenyl ring, and X represents sulphur.

4. Compounds according to Claim 1, wherein alkyl denotes $C_1$–$C_6$-alkyl.

5. Compounds according to Claim 1, wherein alkyl denotes $C_1$–$C_4$-alkyl.

6. Process for the preparation of compounds according to Claim 1 characterised in that
a) substituted anilines of the general formula (II)

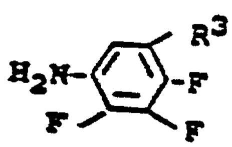

(II)

are reacted with benzoyl iso(thio)cyanates of the general formula (III)

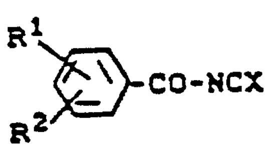

(III)

wherein
X, $R^1$, $R^2$ and $R^3$ have the meaning given in Claim 1 if appropriate in the presence of a diluent, or
b) substituted phenyl iso(thio)cyanates of the general formula (IV)

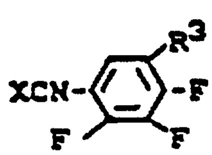

(IV)

are reacted with benzoic acid amides of the general formula (V)

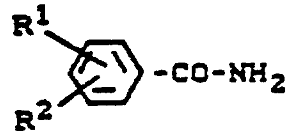

(V)

wherein
X, $R^1$, $R^2$ and $R^3$ have the meanings given in Claim 1, if appropriate in the presence of a catalyst and if appropriate in the presence of a diluent.

7. Agents for combating pests, characterised in that they contain at least one compound of the formula (I) according to Claim 1 or 6.

8. Use of compounds of the formula (I) according to Claim 1 or 6 for combating pests, in particular insects and acarides.

9. Method of combating pests, characterised in that compounds of the formula (I) according to Claim 1 or 6 are allowed to act on the pests, preferably insects or acarides, or their environment.

10. Process for the preparation of agents for combating pests, characterised in that compounds of the formula (I) according to Claim 1 or 6 are mixed with extenders and/or surface-active agents.

**Revendications**

1. Composés de formule générale (I)

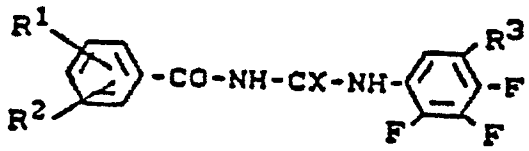

(I)

dans laquelle
X représente l'oxygène ou le soufre,
$R^1$ et $R^2$ sont identiques ou différents et représentent l'hydrogène, un halogène ou un alkyle, et
$R^3$ représente un halogène,
étant entendu que, si
$R^1$ représente le fluor en deuxième position ou le chlore en deuxième position,
$R^2$ représente l'hydrogène, le fluor en sixième position ou le chlore en sixième position et
$R^3$ représente le fluor,
dans ces cas,
X représente le soufre.

2. Composés de formule générale (I) selon la revendication 1, dans laquelle $R^1$ représente l'hydrogène ou un halogène ou un alkyle en deuxième position du cycle benzoylphényle et $R^2$ représente l'hydrogène ou un halogène ou un alkyle en quatrième ou cinquième position du cycle benzoylphényle et X représente l'oxygène.

3. Composés de formule générale (I) selon la revendication 1, dans laquelle $R^1$ représente l'hydrogène ou un halogène ou un alkyle en deuxième position du cycle benzoylphényle et $R^2$ représente l'hydrogène, un halogène ou un alkyle en la quatrième, cinquième ou sixième position du cycle benzoylphényle et X représente le soufre.

4. Composés selon la revendication 1, dans lesquels l'alkyle est un alkyle avec 1 à 6 atomes de carbone.

5. Composés selon la revendication 1, dans lesquels l'alkyle est un alkyle avec 1 à 4 atomes de carbone.

6. Procédé de fabrication de composés selon la revendication 1, caractérisé en ce qu'on fait réagir
a) des anilines substituées de formule générale (II)

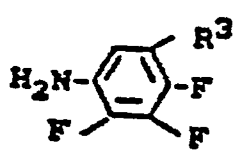

(II)

avec des benzoyl-iso(thio)cyanates de formule générale (III)

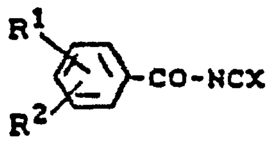
(III)

X, $R^1$, $R^2$ et $R^3$ ayant les significations décrites à la revendication 1,
éventuellement en présence d'un diluant ou
b) des phényl-iso(thio)cyanates substitués de formule générale (IV)

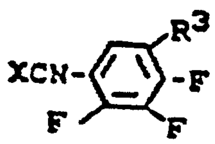
(IV)

avec des amides d'acide benzoïque de formule générale (V)

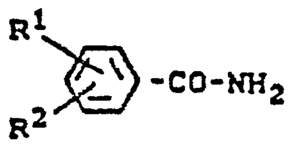
(V)

X, $R^1$, $R^2$ et $R^3$ ayant les significations décrites à la revendication 1,
éventuellement en présence d'un catalyseur et
éventuellement en présence d'un diluant.

7. Agent de lutte contre les parasites, caractérisé en ce qu'il contient au moins un composé de formule (I) selon la revendication 1 ou 6.

8. Utilisation de composés de formule (I) selon la revendication 1 ou 6 pour lutter contre les parasites, en particulier contre les insectes et les acariens.

9. Procédé pour lutter contre les parasites, caractérisé en ce qu'on fait agir les composés de formule (I) selon la revendication 1 ou 6 sur les parasites, de préférence les insectes et les acariens, ou sur leur environnement.

10. Procédé de fabrication d'agents de lutte contre les parasites, caractérisé en ce qu'on mélange les composés de formule (I) selon la revendication 1 ou 6 avec des diluants et/ou des agents tensio-actifs.